# EUROPEAN PATENT APPLICATION

(11) **EP 4 464 774 A1**
(43) Date of publication of application: **20.11.2024**
(21) Application number: 23740452.0
(22) Date of filing: 11.01.2023
(51) Int. Cl.: C12N 5/00, C12N 5/079, C12M 1/12, C07K 14/435, C07K 7/08, C07K 7/06

(54) **CELL CULTURE SCAFFOLD, AND PREPARATION METHOD THEREOF**

(30) Priority: 11.01.2022 KR 20220003931
(71) Applicant: Amolifescience Co., Ltd., Seoul 06527 (KR)
(72) Inventor: SEO, Dong-Sik, Seoul 06527 (KR); IN, Chi-Uk, Seoul 06527 (KR); YOON, Yeo-Joon, Seoul 06527 (KR)
(74) Representative: Weickmann & Weickmann PartmbB
(86) International application number: PCT/KR2023/000514
(87) International publication number: WO 2023/136608

(57) **Abstract**

Provided is a cell culture scaffold. The cell culture scaffold according to an exemplary embodiment of the present invention comprises: a scaffold; and a cell culture coating layer which covers at least a portion of the surface of the scaffold and comprises a fusion polypeptide including a peptide for cell culture and a peptide for adhesion. Accordingly, the cell culture scaffold according to the present invention can achieve high cell culture efficiency due to having excellent adhesiveness with respect to various cells and being capable of stably proliferating adhered cells, can be stored at room temperature for several years, thus having excellent storage stability, prevents imaging interference, which can occur during cell observation or fluorescence analysis on the cell culture scaffold, due to the smooth surface of the cell culture coating layer formed by the fusion polypeptide, thus being applicable to various cell cultures, and, in particular, can express an excellent effect on the proliferation and differentiation of neural stem cells or neural precursor cells.

## Description

### TECHNICAL FIELD

The present invention relates to a cell culture scaffold and a preparation method thereof.

### BACKGROUND

Recently, with the expansion of the use of cultured cells in the treatment of diseases, interest and research on cell culture are increasing. Cell culture is a technology that collects cells from living organisms and cultivates them outside of living organisms, and the cultured cells can be used to treat various diseases by differentiating them into various tissues of the body, such as skin, organs, and nerves, and transplanting them into the human body, or by being transplanted into the human body in a pre-differentiated state to achieve engraftment and differentiation simultaneously.

Culturing mammalian cells is one of many processes in life and health sciences. Cell culture scaffold for mammalian cell culture and analysis involving fixed-dependent cells is often used, for example, in containers such as well-plate made of polymer or glass or plates such as films, which require additional surface treatment that causes the cells to adhere to the surface of the container or plate. Such surface treatment may include forming an adsorption layer on the surface or implementing an appropriate surface shape, for example, by adsorption, grafting or plasma polymerization techniques. Alternatively, the surface treatment may be performed through chemical modification on the container or plate surface itself, and may be performed through atmospheric corona, radio frequency vacuum plasma, DC glow discharge, and microwave plasma treatments or the like.

Meanwhile, nervous system cells can be divided into two main types: central nervous system cells that make up the brain and spinal cord, and peripheral nervous system cells that make up motor nerves, sensory nerves, autonomic nerves or the like. The neurons, astrocytes, and oligodendrocytes that make up the central nervous system (brain and spinal cord) can be made by differentiating neural progenitor cells (NPC) differentiated from pluripotent stem cells, whereas the peripheral nerve cells (motor nerves, autonomic nerves, and sensory nerves) and Schwan cells that make up the peripheral nervous system are derived from neural crest stem cells (NCSC) differentiated from pluripotent stem cells. Therefore, central nervous system cells and peripheral nervous system cells are created along different differentiation pathways from pluripotent stem cells and through neural progenitor cells and neural crest stem cells, respectively, and these different pathways are known to depend on the surrounding environment and the intracellular signaling system.

Diseases of the central nervous system are largely divided into brain disease and spinal cord disease, and diseases caused by abnormal brain function include stroke, dementia, Parkinson's disease or the like. Spinal cord injuries are caused by sudden injuries, and when the spinal cord's function is impaired, problems such as loss of motor control, loss of sensation, and loss of bladder control occur and make the injured live in pain for the rest of their lives. Besides, the number of people suffering from many central nervous system diseases is increasing rapidly as the world ages, and therefore, efforts to treat patients are urgently needed as the quality of life of the patients and their families deteriorates and it places a great social/economic burden on society.

Recently, in order to develop cell replacement therapy, which is considered the most fundamental treatment for central nervous system diseases, research has been actively conducted on how to proliferate or effectively differentiate neural progenitor cells (NPCs), the mother cells of neural stem cells or central nervous system cells.

One of the related studies is that generally, a coating layer using extracellular matrix proteins or several other proteins that help cell proliferation is formed on the surface of a solid scaffold to form a similar microenvironment such as an extracellular matrix to culture and differentiate neural stem cells or neural progenitor cells.

However, the culture scaffold equipped with a coating layer using extracellular matrix proteins or other proteins that help cell proliferation is beneficial for culturing fibroblasts, but is not effective enough to culture and differentiate neural stem cells or neural progenitor cells.

### SUMMARY OF THE INVENTION

### Technical Problem

The present invention has been devised in view of the above problems, and is directed to providing a cell culture scaffold capable of achieving high cell culture efficiency by having excellent cell adhesion and stably proliferating attached cells, and a method for preparing the same.

In addition, the present invention is also directed to providing a cell culture scaffold which can be stored for a long period of several years at room temperature and thus has excellent storage stability, while still maintaining the activity of a substance helpful for cell culture or causing only minimal degradation, thereby enabling cell culture at an initially designed level, and a method for preparing the same.

In addition, the present invention is also directed to providing a cell culture scaffold which can prevent imaging interference, etc., when observing or analyzing fluorescence of cultured cells on a cell culture scaffold due to a substance added to aid in cell culture, and can prevent the cultured cells from being detached from the cell culture scaffold and obtained together with the cultured cells, and a method for preparing the same.

Furthermore, the present invention is also directed to providing a coating composition for cell culture that can achieve the above-mentioned excellent characteristics.

### Technical Solution

In order to solve the above-mentioned problems, the present invention provides a cell culture scaffold, comprising a scaffold, and a cell culture coating layer which covers at least a portion of the surface of the scaffold and comprises a fusion polypeptide comprising a peptide for cell culture and a peptide for adhesion.

According to an exemplary embodiment of the present invention, the scaffold may be a non-porous scaffold formed of one or more materials selected from the group consisting of polycarbonate, polystyrene, polyimide, polyester, polyurethane and glass, or a porous scaffold formed of one or more materials selected from the group consisting of polystyrene (PS), polyester, polyethersulfone (PES), polyvinylidene fluoride (PVDF), polydimethylsiloxane (PDMS), polyamide, polyimide, polyethylene, and polypropylene.

In addition, the peptide for cell culture may have a function of promoting one or more of cell adhesion, movement, proliferation, and differentiation.

In addition, the peptide for adhesion may be derived from a mussel adhesive protein.

In addition, the peptide for adhesion may have any one amino acid sequence selected from the group consisting of SEQ ID NOs: 1 to 15 or one or more amino acid sequences selected from said group which are linked.

In addition, the peptide for cell culture may have any one amino acid sequence selected from the group consisting of SEQ ID NOs: 16 to 27 or one or more amino acid sequences selected from said group which are linked.

In addition, the fusion polypeptide may further include a histidine tag at the N-terminus or C-terminus of the fusion polypeptide.

In addition, the fusion peptide may have an amino acid sequence of SEQ ID NO: 28 or SEQ ID NO: 29.

In addition, the fusion polypeptide may not form granules.

In addition, the scaffold may be of use for culturing neural stem cells or neural progenitor cells.

In addition, the present invention provides a method for preparing a cell culture scaffold, including (1) preparing an active solution including a carbodiimide-based coupling agent and a reactant, and a fusion polypeptide including a peptide for cell culture and a peptide for adhesion; (2) preparing a cell culture coating composition by mixing the prepared active solution and the fusion polypeptide; and (3) forming a cell culture coating layer by treating the cell culture coating composition on the surface of a scaffold.

According to an exemplary embodiment of the present invention, the carbodiimide-based coupling agent may be 1-ethyl-3-(3-dimethylaminopropyl) carbodiimide hydrochloride (EDC) or N,N'-dicyclohexylcarboimide (DCC), and the reactant may be N-hydroxysulfosuccinimide (Sulfo-NHS).

In addition, the carbodiimide coupling agent and the reactant may be contained in the active solution in a weight ratio of 1:0.1 to 10, and in the cell culture coating composition, 1 to 100 parts by weight of the carbodiimide-based coupling agent may be mixed per 100 parts by weight of the fusion polypeptide.

In addition, the present invention provides a coating composition for culturing neural stem cells or neural progenitor cells that forms a cell culture coating layer on the surface of a scaffold, the coating composition for culturing neural stem cells or neural progenitor cells including a fusion polypeptide including a peptide for cell culture and a peptide for adhesion; a carbodiimide-based coupling agent; and a reactant.

Hereinafter, terms used in the present invention will be explained.

The term "extracellular matrix (ECM)" of the present invention refers to a matrix surrounding the outside of a cell, occupying the space between cells, and having a network structure mainly composed of proteins and polysaccharides.

The term "peptide" of the present invention refers to a molecule formed by combining amino acid residues with one another through an amide bond (or peptide bond). The above peptide may be synthesized using genetic recombination and a protein expression system, and may be synthesized in a test tube using, for example, a peptide synthesizer or the like.

The term "fusion polypeptide" of the present invention includes derivatives thereof, and may be fragments in which an amino acid fragment or part of the peptide is substituted or deleted, a part of the amino acid sequence is modified into a structure capable of increasing stability in vivo, a part of the amino acid sequence is modified to increase hydrophilicity, or a part or all of the amino acids are substituted with L- or D-amino acids, or a part of the amino acids is modified.

### Advantageous Effects

The cell culture scaffold according to the present invention has a cell culture coating layer formed of a fusion polypeptide that helps in the culture of various cells, including proliferation and differentiation, and thus has excellent cell adhesion properties and can stably proliferate attached cells, thereby achieving high cell culture efficiency. In addition, despite the fact that the substance that helps in culture such as cell proliferation and differentiation is easily denatured and the storage stability of the polypeptide is unstable, it can be stored for a long period of several years at room temperature, so the storage stability is excellent, and the activity of the polypeptide is maintained as is or only minimally deteriorated, so that cells can be cultured at the initially designed level. In addition, since the surface of the cell culture coating layer formed by the fusion polypeptide is smooth, concerns about imaging interference that may occur during cell observation or fluorescence analysis on the cell culture scaffold can be prevented. Moreover, since the fusion polypeptide is detached from the cell culture coating layer and incorporated into cultured cells, and thus side effects that may occur during various experiments or biotransplantation using cultured cells are prevented, it can be applied to various cell cultures, and in particular, according to one embodiment of the present invention, the cell culture coating layer formed by the preparation method according to the present invention, in which a fusion polypeptide, in which a peptide for cell culture that helps in the proliferation or differentiation of neural stem cells or neural progenitor cells is bonded to a peptide for adhesion, has a synergistic effect in promoting the proliferation and enhancing the differentiation of neural stem cells or neural progenitor cells compared to conventional cell culture coating layers, and thus can be suitable for use in culturing neural stem cells or neural progenitor cells.

### BRIEF DESCRIPTION OF THE DRAWINGS

FIGS. 1A and 1B are optical photomicrographs of the surface of the cell culture scaffold according to Example 1 and Comparative Example 1, respectively.
FIGS. 2A to 2C are optical photomicrographs of the results of producing a neural rosette after culturing pluripotent stem cells on the cell culture scaffold according to Example 2 and Comparative Examples 2 to 3, respectively, and
FIG. 3 is an optical photomicrograph of the results of selecting neural progenitor cells through the cell culture scaffold according to Example 2.

### Best Mode for Carrying Out the Invention

Hereinafter, exemplary embodiments of the present invention will be described in detail so that those of ordinary skill in the art can readily implement the present invention with reference to the accompanying drawings. The present invention may be embodied in many different forms and is not limited to the embodiments set forth herein.

The cell culture scaffold according to an exemplary embodiment of the present invention includes a scaffold and a cell culture coating layer covering at least a part of the surface of the scaffold, and the cell culture coating layer includes a fusion polypeptide including a peptide for cell culture and a peptide for adhesion.

The scaffold is a member for physically supporting a cultured cell, and may be used without limitation in the case of a scaffold that is commonly used in cell culture. In addition, the scaffold may be a non-porous scaffold in whole, or a scaffold in part or in whole part porous. For example, the scaffold may be in the form of a container generally called a well plate, or a non-porous member such as a plate-shaped plate such as a film. In addition, it may be a porous member in which fibers, collectively referred to as nonwoven fabrics or fiber webs, form a three-dimensional network structure or have multiple pores formed in the polymer matrix. Also, these non-porous members or porous members may be laminated or formed as a single layer, but a part may be formed of a non-porous member and the remaining part may be formed of a porous member.

In addition, the scaffold may be used without limitation in the case of a scaffold material used in conventional cell culture, and for example, the non-porous scaffold may be formed of one or more materials selected from the group consisting of polycarbonate, polystyrene, polyimide, polyester, polyurethane, and glass. For example, the porous scaffold may be formed of one or more materials selected from the group consisting of polystyrene (PS), polyester, polyethersulfone (PES), polyvinylidene fluoride (PVDF), polydimethylsiloxane (PDMS), polyamide, polyimide, polyurethane, polyacrylonitrile, polyethylene, and polypropylene.

In addition, the surface on which the cell culture coating layer is to be formed on the scaffold may be subjected to a known surface modification treatment to improve wettability, such as plasma treatment. However, the surface of the scaffold may not be modified to have a functional group for inducing a peptide bond with the fusion polypeptide forming the cell culture coating layer, and accordingly, peptide bonds may not be formed between the surface of the scaffold and the fusion polypeptide, even though an active solution containing a carbodiimide-based coupling agent and a reactant containing N-hydroxysuccinimide is mixed in the cell culture coating composition.

Next, the cell culture coating layer provided on the surface of the above-described scaffold will be described.

The cell culture coating layer is a layer that provides a cell adhesion surface on which cells to be cultured settle and proliferate after being seeded, and is formed through a fusion polypeptide.

The fusion polypeptide comprises a peptide for cell culture and a peptide for adhesion. The peptide for cell culture is a substance that has a function that can help in the culture of cells, particularly neural stem cells or neural progenitor cells, and specifically may be a substance that performs a function of promoting one or more of cell adhesion, movement, proliferation, and differentiation. Known peptides that perform such functions can be used without limitation as the peptide for cell culture. As a non-limiting example, it may include a predetermined amino acid sequence included in any one or more growth factors (GFs) selected from the group consisting of adrenomedullin, angiopoietin, bone morphogenetic protein (BMP), brain-derived neurotrophic factor (BDNF), epidermal growth factor (EGF), erythropoietin, fibroblast growth factor, glial cell line-derived neurotrophic factor (GDNF), granulocyte colony-stimulating factor (G-CSF), granulocyte macrophage colony-stimulating factor (GM-CSF), growth differentiation factor-9 (GDF9), hepatocyte growth factor (HGF), hepatoma-derived growth factor (HDGF), insulin-like growth factor (IGF), keratinocyte growth factor (KGF), migration-stimulating factor (MSF), myostatin (GDF-8), nerve growth factor (NGF), platelet-derived growth factor (PDGF), thrombopoietin (TPO), T-cell growth factor (TCGF), neuropilin, transforming growth factor-alpha (TGF-α), transforming growth factor-beta (TGF-β), tumor necrosis factor-alpha (TNF-α), vascular endothelial growth factor (VEGF), IL-1, IL-2, IL-3, IL-4, IL-5, IL-6 and IL-7. Alternatively, it may include a predetermined amino acid sequence included in any one or more extracellular matrix selected from the group consisting of hyaluronic acid, heparin sulfate, c 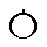 hondroitin sulfate, dermatan sulfate, keratan sulfate, alginate, fibrin, fibrinogen, collagen, elastin, fibronectin, vitronectin, cadherin and laminin. In addition, the peptide for cell culture may be a vitronectin polypeptide, a collagen polypeptide, a laminin polypeptide, a fibronectin polypeptide or a variant thereof.

Preferably, the peptide for cell culture may include an RGD sequence within the amino acid sequence. In addition, the functional peptide may be a peptide having at least one amino acid sequence selected from the group consisting of SEQ ID NO: 16 to SEQ ID NO: 27, or a peptide in which at least one amino acid sequence selected from the group is linked or in which one amino acid sequence selected from the group is repeated two or more times, and through this, it is possible to promote the proliferation of neural stem cells or neural progenitor cells and to exhibit a synergistic effect in enhancing the differentiation efficiency for generating neural rosettes from pluripotent stem cells, thereby enabling the implementation of a cell culture coating layer more suitable for the proliferation and differentiation of neural stem cells or neural progenitor cells. Meanwhile, the effect suitable for proliferation and differentiation of neural stem cells or neural progenitor cells is difficult to view as an effect only of a peptide having at least one amino acid sequence selected from the group consisting of SEQ ID NOs: 16 to 27, or a fusion peptide formed by bonding the peptide with a peptide for adhesion, but may be seen as a synergistic effect that occurs when the above-mentioned fusion peptide forms a cell culture coating layer on the scaffold through the preparation method described below.

Meanwhile, the peptide for cell culture may be, for example, a peptide having 3 to 100 amino acids, more preferably 3 to 50 amino acids, for example 3 to 30 amino acids, and through this, even when stored in a state contained in a coating layer at room temperature for a long period of time, decomposition, denaturation, etc. may be minimized or prevented, which may be more advantageous.

In addition, the peptide for cell culture can be bound to the peptide for adhesion, and specifically, can be bound to the carboxyl terminus, amino terminus, or both the carboxyl terminus and amino terminus of the peptide for adhesion. In this case, the bond may be a covalent bond, and specifically, an amino bond. For example, the fusion polypeptide with SEQ ID NO: 28 is obtained by bonding the peptide for cell culture with SEQ ID NO: 24 and SEQ ID NO: 25, respectively, on both sides of the peptide for adhesion with SEQ ID NO: 15. In addition, for example, the fusion polypeptide with SEQ ID NO: 29 is obtained by providing a histidine tag at the N-terminus of the peptide for adhesion with SEQ ID NO: 13, and bonding a peptide for cell culture with SEQ ID NO: 16 at the C-terminus of the peptide for adhesion with SEQ ID NO: 13. These fusion polypeptides with SEQ ID NOs: 28 and 29 may have excellent effects on the culture, proliferation, and differentiation of neural stem cells and neural progenitor cells compared to other fusion polypeptides.

In addition, peptide for cell culture and peptide for adhesion can be bonded through known methods, and for example, it can be prepared through a recombinant protein production method using E. coli. In addition, it can be directly covalently bonded between peptide for adhesion and peptide for cell culture, but is not limited thereto, and can be connected through a linker. For example, the linker is a peptide linker that will not have specific biological activity other than binding the regions or preserving some minimum distance or other spatial relationships between them, but constituent amino acids can be selected to affect some of the properties of the molecules, such as folding, net charge, or hydrophobicity. Alternatively, it should be noted that a predetermined substance, such as a cross-linking agent, can be used to indirectly connect the peptide for adhesion and the peptide for cell culture.

As mentioned above, the reason why the peptide for cell culture is bound to the peptide for adhesion is because the peptide for adhesion is advantageous for fixing the peptide for cell culture to the surface of the scaffold with good adhesion properties, and has no toxicity that can be applied to culture cells compared to polymer-based adhesive components and is excellent for biocompatibility. In addition, it has the advantage of minimizing desorption after the seeded cells settle on the cell adhesion surface because of its good adhesion properties with the seeded cells.

The peptide for adhesion may be a peptide derived from a mussel adhesive protein, and a known adhesive protein or a variant thereof, collectively referred to as a mussel adhesive protein, may be used without limitation. Preferably, the peptide for adhesion may be a peptide having any one amino acid sequence selected from the group consisting of SEQ ID NOs: 1 to 15, a peptide in which one or more amino acid sequences selected from the group are linked, or a peptide in which one amino acid sequence selected from the group is repeated twice or more.

Meanwhile, the cell culture coating layer formed with the above-described fusion polypeptide may have a form in which granules in which the cell culture fusion polypeptide is formed are aggregated due to the peptide for adhesion derived from the mussel adhesive protein and the cell culture coating layer forming method described below. Alternatively, in the case of a scaffold made of a porous member, the scaffold may be implemented in a form in which the space between the fibers exposed on the surface of the porous member is linked by a membrane. In the case of a cell culture coating layer having this form, there is an advantage in that the cell culture efficiency is very excellent, and at the same time, even when stored at room temperature for several years or more, the activity decrease of the peptide for cell culture caused by the decomposition or denaturation of the fusion polypeptide for cell culture forming the cell culture coating layer is prevented or minimized, thereby greatly improving the storage stability.

However, there is a concern that the surface of the cell culture coating layer formed by coating the fusion polypeptide in the form of granules or membranes may interfere with observation and analysis when observing cells cultured on a cell culture scaffold or analyzing them using fluorescent staining. In addition, there is a concern that the granular or film-shaped coating portion formed by the fusion polypeptide may detach from the cell culture coating layer and be included in the cultured cells as an impurity when separating the cultured cells from the cell culture scaffold. Accordingly, according to one embodiment of the present invention, by controlling the difference in reaction rate due to the amino acid sequence of the fusion polypeptide and the composition of the active solution, the degree to which the fusion peptide forms a film on the surface of a grain or porous member can be controlled or without being formed, so that a cell culture coating layer having a smooth surface is formed. As an example of this, in order to control the reaction rate with the fusion polypeptide through the composition of the active solution, as described below, the reactant provided in the active solution is made to contain N-hydroxysuccinimide, and preferably, the reactant does not contain N-hydroxysulfosuccinimide, thereby implementing a cell culture coating layer in the form of aggregated grains or, in the case of a porous scaffold, in the form of a coating film that blocks the pores of the fiber surface, so that the surface of the scaffold is smoothly covered. Alternatively, as another example, a cell culture coating layer having a smooth surface can be implemented by controlling the reaction rate with an active solution through the amino acid sequence of the fusion polypeptide. As a specific example of this, in the case of a fusion peptide (Ex. SEQ ID NO: 28) implemented using a peptide for adhesion with SEQ ID NO: 2 or SEQ ID NO: 15, even when N-hydroxysulfosuccinimide is used as a reactant of an active solution, a cell culture coating layer having a smooth surface without forming granules can be implemented.

In addition, the fusion polypeptide may further include a histidine tag at the N-terminus or C-terminus of the fusion polypeptide in order to confirm whether the fusion polypeptide is contained in cells obtained by separating the fusion polypeptide from the scaffold after culture or to isolate the fusion polypeptide from the obtained cells. The histidine tag has an amino acid sequence of 3 to 10 histidines linked together, and may have an amino acid sequence of 6 histidines linked together, for example.

In addition, according to one embodiment of the present invention, the cell culture coating layer has a smooth surface without forming grains, and as described above, the cell culture coating composition contains N-hydroxysuccinimide as a reactant to have such a surface shape, so that N-hydroxysuccinimide can be detected in the finally implemented cell culture coating layer. Although N-hydroxysuccinimide is washed away through multiple washing processes after the production of the cell culture coating layer, trace amounts may remain in the cell culture coating layer and thus may be detected in the final implemented cell culture coating layer. The detection of the N-hydroxysuccinimide can be performed using a known method and conditions capable of detecting N-hydroxysuccinimide, and for example, the presence or absence of N-hydroxysuccinimide can be detected by comparing the measured values of N-hydroxysuccinimide known through ¹H-NMR, ¹³C-NMR, ¹⁵N-NMR, mass spectrum (MS spectrum), Raman spectrum, FTIR, etc. with the measured values in the cell culture coating layer. Specifically, when the cell culture coating layer has a peak value at a corresponding position according to the detection method below, it can be determined that N-hydroxysuccinimide is present. For example, it may have peaks at 2.7 ppm ± 0.05 ppm and/or 2.56 ppm ± 0.05 ppm relative to TMS in the ¹H solid-state NMR spectrum, it may have a peak at 25.5 ppm ± 0.05 ppm relative to TMS in the ¹³C solid-state NMR spectrum, and/or it may have a peak at -167 ppm ± 0.05 ppm relative to TMS in the ¹⁵N solid-state NMR spectrum. In addition, it may have peaks at 28 m/z, 55 m/z, and 115 m/z in the mass spectrum (MS spectrum). In addition, it may have a peak value at 725 cm⁻¹±5.0 cm⁻¹ in the Raman spectrum.

A cell culture scaffold according to one embodiment of the present invention, wherein the cell culture coating layer described above is provided on the surface of the scaffold, can be prepared by including (1) a step of preparing an active solution containing a carbodiimide-based coupling agent and a reactant containing N-hydroxysuccinimide, and a fusion polypeptide including a peptide for cell culture and a peptide for adhesion, (2) a step of preparing a cell culture coating composition by mixing the prepared active solution and the fusion polypeptide, and (3) a step of forming a cell culture coating layer by treating the cell culture coating composition on the surface of a scaffold.

First, as step (1) according to the present invention, a step of preparing an active solution containing a carbodiimide-based coupling agent and a reactant containing N-hydroxysuccinimide, and a fusion polypeptide including a peptide for cell culture and a peptide for adhesion, respectively, is performed.

The active solution contains a carbodiimide-based coupling agent and a reactant containing N-hydroxysuccinimide, and may further contain a solvent. The active solution is a substance that introduces the fusion polypeptide to the surface of the scaffold, and, compared to the case where the fusion polypeptide is simply treated on the surface of the scaffold by a conventional method, it improves the adhesion between the cell culture coating layer and the surface of the scaffold, and has the advantage of reducing the risk of infection by external bacteria in the state where the fusion polypeptide is introduced to the scaffold, enabling stable long-term storage even under temperature changes, and allowing the cultured cells to be cultured with less variation in size, differentiation level, etc. during cell culture. Meanwhile, the active solution plays a role in introducing the fusion polypeptide to the surface of the scaffold, but the introduction at this time does not mean introducing by inducing a peptide bond between the fusion polypeptide and the carboxyl group and/or amine group provided on the surface of the scaffold as described above.

A coupling agent that allows fusion polypeptides to be linked to each other may be used without limitation as the carbodiimide-based coupling agent, and it may be, for example, 1-[3-(dimethylamino)propyl]-3-ethylcarboimide hydrochloride (EDC) or N,N'-dicyclohexylcarboimide (DCC).

In addition, the reactant is provided to increase the efficiency of fusion polypeptides binding to each other by preventing hydration of fusion proteins coupled to carbodiimide-based coupling agents, and contains N-hydroxysuccinimide. However, due to the amino acid sequence of the fusion polypeptide, the reactant may not contain N-hydroxysulfosuccinimide (Sulfo-NHS) to reduce the effect on the reaction rate with the active solution, and this may be more advantageous in forming a cell culture coating layer having a smooth surface on the scaffold surface without considering the amino acid sequence of the fusion polypeptide by preventing the formation aspect of a cell culture coating layer, that is, the formation of granules or the formation of a coating film on the surface of a scaffold that is porous member, which occurs due to the inclusion of N-hydroxysulfosuccinimide in the reactant.

The active solution may contain the carbodiimide-based coupling agent and the reactant in a weight ratio of 1:0.1 to 10. If these are not included in an appropriate ratio, it is difficult to achieve the effect intended by the present invention, and there is a concern that the adhesion of cells in the implemented cell culture coating layer may be significantly reduced.

In addition, the active solution may further contain sodium acetate to enhance reactivity. In this case, the sodium acetate may be included in an amount of 1 to 100 parts by weight per 100 parts by weight of the carbodiimide-based coupling agent.

In addition, the active solution may further include a solvent, which may be water or an organic solvent, for example water.

The method for preparing the active solution is not particularly limited, but as an example, two types of solutions may be prepared by adding a sodium acetate solution to a carbodiimide-based coupling agent solution and a reactant solution respectively and mixing them, then mixing the two types of solutions in an appropriate ratio and inducing a reaction for 30 to 60 minutes, and then reacting again for 25 to 40 minutes in a constant temperature incubator at 28 to 35 °C, thereby preparing the final active solution.

Next, as step (2) according to the present invention, a step of preparing a cell culture coating composition by mixing the prepared active solution and the fusion polypeptide is performed.

In this case, the fusion polypeptide and the active solution may be mixed by adjusting the content so that 1 to 100 parts by weight of the carbodiimide-based coupling agent is included per 100 parts by weight of the fusion polypeptide for cell culture. If the carbodiimide-based coupling agent is less than 1 part by weight, cells may not be adhered or may differentiate, and if it exceeds 100 parts by weight, cells may detach after adhesion, making it difficult to stably culture cells.

In addition, the prepared active solution and the fusion polypeptide may be prepared into a final cell culture coating composition after inducing a reaction for 0 to 2 hours after mixing.

Next, as step (3) according to the present invention, a step of forming a cell culture coating layer by treating the cell culture coating composition on the surface of a scaffold is performed.

The method for treating the prepared cell culture coating composition on the surface of the scaffold may be a conventional coating method, and for example, in the case of a scaffold such as a well plate, it may be dispensed using a pipette aid. After treating the cell culture coating composition on the surface, a reaction may be induced in a constant temperature incubator at 4 to 60 °C for 0 minutes to 2 hours to form a cell culture coating layer.

Thereafter, it may be further subjected to a washing process, and for example, the washing process may be repeated 2 to 5 times in total, with more than 0 to 30 minutes using tertiary distilled water. After going through the washing process, it may be naturally dried in air, thereby preparing a cell culture scaffold.

Table 1 below shows amino acid sequences for the above-described peptide for adhesion and peptide for cell culture.

**[Table 1]**

| SEQ ID NO | Amino Acid Sequence |
|---|---|
| 1 | Ala Lys Pro Ser Tyr Pro Pro Thr Tyr Lys |
| 2 | Ala Lys Pro Ser Tyr Pro Pro Thr Tyr Lys Ala Lys Pro Ser Tyr Pro Pro Thr Tyr Lys |
| 3 | Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys |
| 4 | Glu Val His Ala Cys Lys Pro Asn Pro Cys Lys Asn Asn Gly Arg Cys Tyr Pro Asp Gly Lys Thr Gly Tyr Lys Cys Lys Cys Val Gly Gly Tyr Ser Gly Pro Thr Cys Ala Cys |
| 5 | Ala Asp Tyr Tyr Gly Pro Lys Tyr Gly Pro Pro Arg Arg Tyr Gly Gly Gly Asn Tyr Asn Arg Tyr Gly Gly Ser Arg Arg Tyr Gly Gly Tyr Lys Gly Trp Asn Asn Gly Trp Lys Arg Gly Arg Trp Gly Arg Lys Tyr Tyr Glu Phe Glu Phe |
| 6 | Ala Asp Tyr Tyr Gly Pro Lys Tyr Gly Pro Pro Arg Arg Tyr Gly Gly Gly Asn Tyr Asn Arg Tyr Gly Arg Arg Tyr Gly Gly Tyr Lys Gly Trp Asn Asn Gly Trp Lys Arg Gly Arg Trp Gly Arg Lys Tyr Tyr |
| 7 | Gly His Val His Arg His Arg Val Leu His Lys His Val His Asn His Arg Val Leu His Lys His Leu His Lys His Gln Val Leu His Gly His Val His Arg His Gln Val Leu His Lys His Val His Asn His Arg Val Leu His Lys His Leu His Lys His Gln Val Leu His |
| 8 | Ser Ser Glu Glu Tyr Lys Gly Gly Tyr Tyr Pro Gly Asn Ala Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Lys Tyr Tyr Gly Gly Ser Ser |
| 9 | Ser Ser Glu Glu Tyr Lys Gly Gly Tyr Tyr Pro Gly Asn Thr Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Lys Tyr Tyr Gly Gly |
| | Gly Ser Ser |
| 10 | Tyr Asp Asp Tyr Ser Asp Gly Tyr Tyr Pro Gly Ser Ala Tyr Asn Tyr Pro Ser Gly Ser His Trp His Gly His Gly Tyr Lys Gly Lys Tyr Tyr Gly Lys Gly Lys Lys Tyr Tyr Tyr Lys Phe Lys Arg Thr Gly Lys Tyr Lys Tyr Leu Lys Lys Ala Arg Lys Tyr His Arg Lys Gly Tyr Lys Lys His Tyr Gly Gly Ser Ser Ser |
| 11 | Ser Ser Glu Glu Tyr Lys Gly Gly Tyr Tyr Pro Gly Asn Thr Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Lys Tyr Tyr Gly Gly Gly Ser Ser |
| 12 | Gly Gly Gly Asn Tyr Arg Gly Tyr Cys Ser Asn Lys Gly Cys Arg Ser Gly Tyr Ile Phe Tyr Asp Asn Arg Gly Phe Cys Lys Tyr Gly Ser Ser Ser Tyr Lys Tyr Asp Cys Gly Asn Tyr Ala Gly Cys Cys Leu Pro Arg Asn Pro Tyr Gly Arg Val Lys Tyr Tyr Cys Thr Lys Lys Tyr Ser Cys Pro Asp Asp Phe Tyr Tyr Tyr Asn Asn Lys Gly Tyr Tyr Tyr Tyr Asn Asp Lys Asp Tyr Phe Asn Cys Gly Ser Tyr Asn Gly Cys Cys Leu Arg Ser Gly Tyr |
| 13 | Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ser Ser Glu Glu Tyr Lys Gly Gly Tyr Tyr Pro Gly Asn Ala Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys |
| | Tyr Lys Tyr Leu Lys Lys Ala Arg Lys Tyr His Arg Lys Gly Tyr Lys Tyr Tyr Gly Gly Ser Ser Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys |
| 14 | Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ser Ser Glu Glu Tyr Lys Gly Gly Tyr Tyr Pro Gly Asn Thr Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Lys Tyr Tyr Gly Gly Gly Ser Ser Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys |
| 15 | Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys |
| 16 | Lys Gly Gly Pro Gln Cys Val Thr Arg Gly Asp Val Phe Cys Thr Pro |
| 17 | Gly Ala Cys Arg Gly Asp Cys Leu Gly Ala |
| 18 | Ser Glu Thr Gln Arg Gly Asp Val Phe Val Pro Met |
| 19 | Pro Met Gln Lys Met Arg Gly Asp Val Phe Ser Pro |
| 20 | Glu Ala Pro Arg Gly Asp Val Tyr Gln Gly |
| 21 | Ser Glu Cys Thr Gln Arg Gly Asp Val Phe Cys Val Pro Met |
| 22 | Pro Met Gln Cys Lys Met Arg Gly Asp Val Phe Cys Ser Pro |
| 23 | Glu Cys Ala Pro Arg Gly Asp Val Tyr Cys Gln Gly |
| 24 | Gly Pro Gln Cys Val Thr Arg Gly Asp Val Phe Cys Thr Pro |
| 25 | Gly Gly Gly Pro Gln Cys Val Thr Arg Gly Asp Val Phe Cys Thr Pro |
| 26 | Gly Arg Gly Asp Ser Pro |
| 27 | Lys Ser Ser Pro His Ser Arg Asn Ser Gly Ser Gly Ser Gly Ser Gly Ser Gly Arg Gly Asp Ser Pro |
| 28 | Gly Pro Gln Cys Val Thr Arg Gly Asp Val Phe Cys Thr Pro Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Gly Gly Gly Pro Gln Cys Val Thr Arg Gly Asp Val Phe Cys Thr Pro |
| 29 | His His His His His His Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ser Ser Glu Glu Tyr Lys Gly Gly Tyr Tyr Pro Gly Asn Ala Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr |
| | Lys Asn Ser Gly Lys Tyr Lys Tyr Leu Lys Lys Ala Arg Lys Tyr His Arg Lys Gly Tyr Lys Tyr Tyr Gly Gly Ser Ser Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Lys Gly Gly Pro Gln Cys Val Thr Arg Gly Asp Val Phe Cys Thr Pro |

### Mode for Carrying Out the Invention

The present invention will be described in more detail through the following examples, but the following examples are not intended to limit the scope of the present invention, which should be construed to aid understanding of the present invention.

### <Example 1>

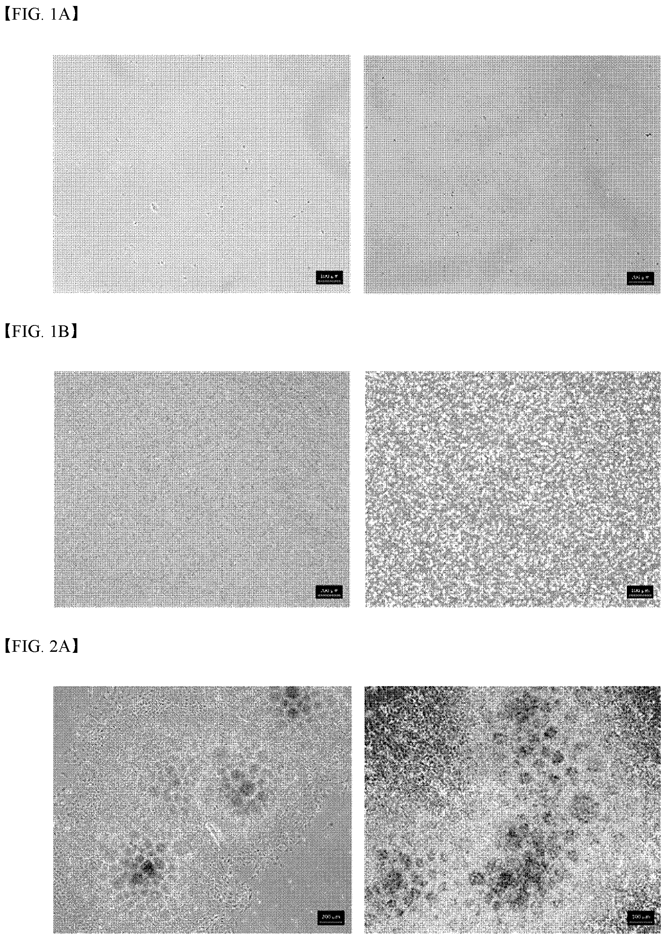

A 6-well plate made of sterilized polystyrene material was prepared as a substrate. In this case, a 6-well plate without plasma treatment was prepared. Afterwards, 2 ml of the cell culture coating composition prepared in the following preparation example was dispensed into each well using a pipette aid, and the mixture was reacted in a constant temperature incubator to form a cell culture coating layer on the surface of the substrate. Afterwards, the plate was washed three times for 10 minutes each using tertiary distilled water, and then dried in the air with the plate lid open in a clean bench to prepare a cell culture scaffold having a cell culture coating layer having a smooth surface as shown in FIG. 1A.

### * Preparation Example - Preparation of Cell Culture Coating Composition

A fusion polypeptide was prepared having an amino acid sequence of SEQ ID NO: 29. In this case, the fusion polypeptide was prepared by a recombinant protein production method using E. coli.

Meanwhile, to prepare an active solution, a solution of NaOAc, NaHCO₃, and 2-(N-morpholino)ethanesulfonic acid dissolved in tertiary distilled water was first prepared, and then each was placed into a microtube containing EDC and N-hydroxysuccinimide reagent as a reactant, to prepare an EDC solution and an N-hydroxysuccinimide (hereinafter referred to as 'NHS') solution.

To prepare a cell culture coating composition, an EDC solution was poured into a conical tube, then an NHS solution was added and stirred, and 0.1 mg of a fusion polypeptide for cell culture was added to the prepared active solution and stirred to prepare a cell culture coating composition. In this case, the cell culture coating composition contained 1 part by weight of EDC per 100 parts by weight of the fusion polypeptide, and EDC and NHS were mixed at a weight ratio of 1:2, and NaOAc included in the coating composition was included 100 parts by weight per 100 parts by weight of EDC. At this time, the concentration of the fusion protein for cell culture in the cell culture coating composition was 0.05 mg/ml.

### <Comparative Example 1>

A cell culture scaffold was prepared in the same manner as in Example 1, except that the type of the reactant in the active solution of the cell culture coating composition was changed from N-hydroxysuccinimide to N-hydroxysulfosuccinimide, so that the fusion polypeptide for cell culture, as in FIG. 1B, formed granules and cell culture scaffold having a cell culture coating layer formed by the aggregation of granules was prepared.

### <Experimental Example 1>

Embryonic stem cells were dispensed in the same amount on the cell culture scaffolds according to Example 1 and Comparative Example 1, and then cultured for 7 days using mTeSR culture medium, and the cultured results were photographed using an optical microscope. Afterwards, 10 researchers in related technical fields equally evaluated the observation items for the shape of the cultured cells through the photographs taken, and the opinions of the 10 researchers were collected regarding the ease of cell observation during the evaluation.

As a result of the opinion collection, regarding the ease of cell observation, 8 out of 10 researchers evaluated that the cell culture scaffold according to Example 1 was easier to observe cultured cells than the cell culture scaffold according to Comparative Example 1, and 2 evaluated that they were similar.

These evaluation results show that the surface roughness of the cell culture coating layer can affect the observation of cultured cells, and that the cell culture scaffold according to Example 1 is more advantageous for cell observation than Comparative Example 1.

### <Example 2>

A cell culture scaffold was manufactured in the same manner as in Example 1, but the fusion polypeptide was changed to one having SEQ ID NO: 28.

### <Comparative Example 2>

A cell culture scaffold was prepared in the same manner as in Example 2, but instead of the fusion polypeptide having the SEQ ID NO: 28, a peptide for cell culture having the SEQ ID NO: 25 corresponding to the peptide for cell culture included in the fusion polypeptide having the SEQ ID NO: 28 was used to prepare the cell culture scaffold.

### <Comparative Example 3>

A cell culture scaffold was prepared by coating Matrigel, a commercially available coating composition for cell culture, on a 6-well plate, as a substrate, made of sterilized polystyrene material, according to the protocol of the manufacturer of the coating composition.

### <Experimental Example 2>

The following physical properties were evaluated for the cell culture scaffolds according to Example 2 and Comparative Examples 2 to 3.

### 1. Evaluation of differentiation efficiency of pluripotent stem cells

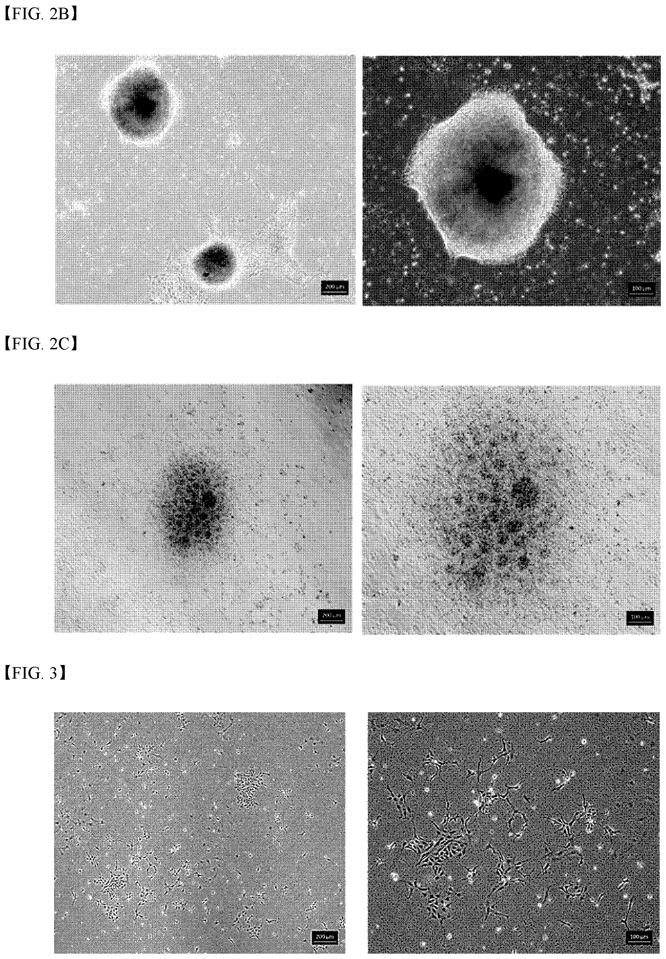

Human embryonic stem cells were seeded on low attachment plates and cultured for 7 days in EB culture medium (DMEM/F 12, kSR, NEAA, b-ME, SB431542, Dorsomorphin, 0.5% penicillin-streptomycin) to obtain EBs (Embryoid bodies). The obtained EBs were seeded on a cell culture scaffold, and the culture medium was exchanged every 2 to 3 days in N2 culture medium (DMEM/F12, N2, insulin, bFGF), passaged every 5 days, and the differentiation results were observed using an optical microscope, and the results are shown in FIG. 2A (Example 2), FIG. 2B (Comparative Example 2), and FIG. 2C (Comparative Example 3), respectively.

As can be seen in FIG. 2A, the cell culture scaffold according to Example 2 had excellent neural rosette formation properties since it included a cell culture coating layer formed of a fusion polypeptide having SEQ ID NO: 28, and it can be seen that neural rosette formation was significantly superior to that of Comparative Example 3 (FIG. 2C) which had a cell culture coating layer coated with a commercial cell culture coating material.

Meanwhile, it can be seen that the cell culture scaffold of Comparative Example 2, which is FIG. 2B, has less neural rosette formation than Example 2 because the peptide for adhesion is not fused, even though the same peptide for cell culture as the fusion polypeptide used in Example 2 was used.

### 2. Neural progenitor cell selection

After seeding neural rosettes on the cell culture scaffold according to Example 2, the culture medium was exchanged every 2 to 3 days in N2 culture medium (DMEM/F12, N2, insulin, bFGF), passaged every 6 to 7 days, and neural progenitor cells were selected, and the optical microscope photograph thereof is shown in FIG. 3.

As can be seen in FIG. 3, the transformation from neural rosettes to neural progenitor cells was confirmed through the cell scaffold according to Example 2.

### <Comparative Example 4>

A cell culture scaffold was prepared by coating Vitronectin-XF^{™}, a commercially available coating composition for cell culture, on a 6-well plate, as a substrate, made of sterilized polystyrene material, according to the protocol of the manufacturer of the coating composition.

### <Experimental Example 3>

Accelerated aging tests were performed on the cell culture scaffolds according to Example 2 and Comparative Examples 3 to 4 according to the guidelines based on the Criteria for Establishment of Shelf Life and Stability Evaluation of Medical Devices pursuant to the Notice No. 2014-178 of the Ministry of Food and Drug Safety of the Republic of Korea (revised on October 31, 2014), and then neural stem cells were cultured to evaluate their storage stability.

Specifically, to reproduce real-time aging of cell culture scaffolds within a shortened time, the cell culture scaffolds were stored at an elevated temperature (60 °C) for 0 and 3 months, and the aging periods of each cell culture scaffold were prepared to be 0 years and 3 years, respectively.

For each example and comparative example, the same amount of embryonic stem cells was dispensed onto each prepared cell culture scaffold, and then cultured for 6 days using mTeSR culture medium, and was photographed with an optical microscope to observe whether cell culture was successful, and the number of cultured cells was counted, and based on the 0-month counting result of Example 1 as 100%, the remaining experimental results are presented as relative percentages in Table 2 below.

**[Table 2]**

| | Number of cultured cells per accelerated specimen (%) | |
|---|---|---|
| | 0 months | 3 months |
| Example 2 | 100 | 104.6 |
| Comparative Example 3 | 98.1 | 0 |
| Comparative Example 4 | 95.5 | 12.4 |

As can be seen in Table 2,
In the case of the substrate of the cell culture scaffold according to Example 2, it can be seen that even when the aging period was accelerated to 3 years, the cell culture efficiency was not reduced compared to the case where aging was not performed. However, in the case of Comparative Examples 3 and 4, when the aging period is 3 years, the cell culture performance is significantly reduced or not expressed compared to 0 years, so it can be expected that the storage stability is not good.

Although exemplary embodiments of the present invention have been described above, the idea of the present invention is not limited to the embodiments set forth herein. Those of ordinary skill in the art who understand the idea of the present invention may easily propose other embodiments through supplement, change, removal, addition, etc. of elements within the scope of the same idea, but the embodiments will be also within the idea scope of the present invention.

[Sequence Listing Free Text]

## Claims

1. A cell culture scaffold, comprising:
a scaffold; and
a cell culture coating layer which covers at least a portion of the surface of the scaffold and comprises a fusion polypeptide comprising a peptide for cell culture and a peptide for adhesion.

2. The cell culture scaffold of claim 1,
wherein the scaffold is:
a non-porous scaffold formed of one or more materials selected from the group consisting of polycarbonate, polystyrene, polyimide, polyester, polyurethane and glass, or
a porous scaffold formed of one or more materials selected from the group consisting of polystyrene (PS), polyester, polyethersulfone (PES), polyvinylidene fluoride (PVDF), polydimethylsiloxane (PDMS), polyamide, polyimide, polyurethane, polyacrylonitrile, polyethylene, and polypropylene.

3. The cell culture scaffold of claim 1, wherein the peptide for cell culture has a function of promoting one or more of cell adhesion, movement, proliferation, and differentiation.

4. The cell culture scaffold of claim 1, wherein the peptide for adhesion is derived from a mussel adhesive protein.

5. The cell culture scaffold of claim 1, wherein the peptide for adhesion has any one amino acid sequence selected from the group consisting of SEQ ID NOs: 1 to 15 or one or more amino acid sequences selected from said group which are linked.

6. The cell culture scaffold of claim 1, wherein the peptide for cell culture has any one amino acid sequence selected from the group consisting of SEQ ID NOs: 16 to 27 or one or more amino acid sequences selected from said group which are linked.

7. The cell culture scaffold of claim 1, wherein the fusion polypeptide further comprises a histidine tag at the N-terminus or C-terminus of the fusion polypeptide.

8. The cell culture scaffold of claim 1, wherein the fusion polypeptide has an amino acid sequence of SEQ ID NO: 28 or SEQ ID NO: 29.

9. The cell culture scaffold of claim 1, wherein the fusion polypeptide forms a cell culture coating layer without forming granules.

10. The cell culture scaffold of claim 1, for use in culturing neural stem cells or neural progenitor cells.

11. The cell culture scaffold of claim 1, wherein no peptide bond is formed between the scaffold and the fusion polypeptide.

12. A method for preparing a cell culture scaffold, comprising:
(1) preparing an active solution comprising a carbodiimide-based coupling agent and a reactant containing N-hydroxysuccinimide, and a fusion polypeptide comprising a peptide for cell culture and a peptide for adhesion;
(2) preparing a cell culture coating composition by mixing the prepared active solution and the fusion polypeptide; and
(3) forming a cell culture coating layer by treating the cell culture coating composition on the surface of a scaffold.

13. The method for preparing a cell culture scaffold of claim 12, wherein the carbodiimide-based coupling agent is 1-ethyl-3-(3-dimethylaminopropyl) carbodiimide hydrochloride (EDC) or N,N'-dicyclohexylcarboimide (DCC), and the reactant does not contain N-hydroxysulfosuccinimide.

14. The method for preparing a cell culture scaffold of claim 12,
wherein the carbodiimide coupling agent and the reactant are contained in the active solution in a weight ratio of 1:0.1 to 10, and
in the cell culture coating composition, 1 to 100 parts by weight of the carbodiimide-based coupling agent is mixed per 100 parts by weight of the fusion polypeptide.

15. A coating composition for culturing neural stem cells or neural progenitor cells that forms a cell culture coating layer on the surface of a scaffold, the coating composition for culturing neural stem cells or neural progenitor cells comprising:
a fusion polypeptide comprising a peptide for cell culture and a peptide for adhesion;
a carbodiimide-based coupling agent; and
a reactant containing N-hydroxysuccinimide.
